# NOUVEAU FASCICULE DE BREVET EUROPEEN

(11) **EP 0 318 369 B2**
(45) Date de publication et mention de la décision concernant l'opposition: **22.03.1995**
(45) Mention de la délivrance du brevet: 29.01.1992
(21) Numéro de dépôt: 88402921.6
(22) Date de dépôt: 22.11.1988
(51) Int. Cl.: A61K 7/00, A61K 7/42, A61K 7/13

(54) **Composition à base de phases lamellaires lipidiques hydratées ou de liposomes contenant de la tyrosine ou un dérivé de tyrosine et composition cosmétique ou pharmaceutique, notamment dermatologique, à activité pigmentante, l'incorporant**
Zusammensetzung aus lipiden lamellaren Hydratwasser enthaltenden Phasen oder Liposomen, die Tyrosin oder ein Tyrosinderivat enthalten und kosmetische oder pharmazeutische, insbesondere dermatologische Zusammensetzung mit pigmentierender Wirkung
Hydrated lipidic lamellar composition or liposome containing tyrosine or tyrosine derivative and cosmetic or pharmaceutical topical tanning composition thereof

(30) Priorité: 27.11.1987 FR 8716524
(43) Date de publication de la demande: 31.05.1989
(73) Titulaire: LVMH RECHERCHE, 92752 Nanterre (FR)
(72) Inventeur: Meybeck, Alain, F-92400 Colombes (FR); Dumas, Marc, F-92700 Colombes (FR)
(74) Mandataire: Portal, Gérard

(56) Documents cités:
- EP-A- 0 070 048
- EP-A- 0 107 559
- EP-A- 0 241 573
- FR-A- 2 408 387
- FR-A- 2 416 008
- GB-A- 2 013 609
- GB-A- 2 176 481
- US-A- 3 957 971
- US-A- 4 217 344
- J. Soc. Cosmet. Chem. 28, 285-314 (1977)
- Hawley's Condensed Chem. Dictionary, Van Nostrand Reinhold Cy, p. 1202 (1987)
- The Merck Index, p. 1406 (1983)

## Description

La présente invention a pour objet une composition à base de phases lamellaires lipidiques hydratées ou de liposomes, contenant de la tyrosine ou un dérivé de la tyrosine.

L'invention trouve notamment application dans le domaine cosmétique ou pharmaceutique, notamment dermatologique, pour la réalisation de compositions à activité pigmentante, pouvant être utilisées pour accélérer le bronzage de la peau, améliorer le teint ou ralentir l'apparition des cheveux gris.

On sait que les pigments mélaniques, responsables en particulier de la couleur noire de la peau et des cheveux, ont une structure macromoléculaire, dont les unités chimiques prédominantes sont formées à partir de précurseurs, et notamment à partir de la tyrosine.

L'article de PRAKASH C. JOSHI et al., Biochem. Biophys. Res. Commun (1987) 142(1) pages 265 à 274, rappelle le mécanisme biochimique de transformation de la tyrosine en mélanine.

On a donc cherché depuis longtemps à développer des produits cosmétiques à base de tyrosine, destinés notamment à favoriser le bronzage de la peau, et la pigmentation des cheveux.

Cependant, la tyrosine présente un certain caractère hydrophile et pénètre très difficilement au travers de la couche cornée jusqu'au niveau des mélanocytes. De ce fait, la recherche s'est orientée vers des produits dans lesquels la tyrosine est présente sous une forme modifiée, tant du point de vue chimique que physique.

C'est ainsi que le brevet français FR-A-2439013 préconise l'utilisation d'un sel complexe de L-tyrosine avec au moins un acide aminé choisi parmi l'arginine, l'ornithine, la citrulline, la lysine ou l'hydroxylysine. Les auteurs ont montré que le sel complexe précité a un effet inattendu d'activation de la tyrosinase naturellement présente dans l'épiderme.

De même, le document DE-2932923 révèle des compositions cosmétiques contenant un complexe tyrosine-arginine-urocanate, agissant comme promoteur de mélanine.

On a également développé des compositions cosmétiques incorporant des associations de composées variés, parmi lesquels figure la tyrosine (FR-A-2252841 et US-3899288).

De même, le document EP-124077 révèle une composition destinée à ralentir l'apparition des cheveux gris. Il y est indiqué que les résultats obtenus avec cette composition sont meilleurs si on utilise préalablement à celle-ci une solution contenant de la tyrosine.

Enfin, le brevet US-4453941 décrit l'utilisation d'un dérivé spécifique de la tyrosine, possédant une chaîne latérale susceptible de former un noyau indole, en association avec un acide aminé et un oxydant.

Il existe donc depuis longtemps un préjugé à l'encontre de l'utilisation de la tyrosine seule, dans des compositions cosmétiques.

Ce préjugé a été confirmé récemment par un article de C. JAWORSKI et al., publié dans "Journal of the American Academy of Dermatology" 1987, vol 16, 4, pages 769-771. Les auteurs n'ont observé aucun effet significatif des compositions testées contenant de la tyrosine, sur l'accélération du bronzage de la peau. Ils font remarquer en outre qu'il serait douteux que la tyrosine, molécule polaire, puisse traverser le stratum corneum et pénétrer ainsi jusqu'à la couche basale de l'épiderme où se situent les mélanocytes.

Le document US-A-3357971 cite de manière fortuite la présence de tyrosine dans une formulation SPIER-PASCHER de substances hydrosolubles, la formulation SPIER-PASCHER ayant un effet hydratant.

On connaît également depuis longtemps l'emploi de phases lamellaires lipidiques hydratées ou de liposomes dans des compositions pharmaceutiques ou des compositions cosmétiques, dans lesquelles on incorpore divers principes actifs (EP-B1-0087993 ou US-A-4217344 ou FR-A-2408387).

A l'encontre du préjugé existant, les auteurs de la présente invention ont mené des recherches et mis en évidence, de façon tout à fait inattendue, le fait que la tyrosine ou ses dérivés, tels que ses sels ou ses esters, lorsqu'ils sont incorporés dans une phase lamellaire lipidique hydratée ou dans des liposomes, permettent d'obtenir une augmentation mesurable de la quantité de mélanine induite dans la peau par les rayons ultra-violets. Cette découverte rend possible la réalisation de compositions cosmétiques ou pharmaceutiques incorporant de la tyrosine, ou l'un de ses dérivés.

On peut ainsi en déduire en quelque sorte un effet de potentialisation de l'activité de la tyrosine ou de ses dérivai, par leur incorporation dans des phases lamellaires lipidiques hydratées ou dans des liposomes.

Ainsi, la présente invention a pour effet de résoudre le nouveau problème technique consistant en la fourniture d'une nouvelle formulation à base de tyrosine ou de l'un de ses dérivés, tels que ses sels ou ses esters, permettant de potentialiser leur efficacité, pour permettre leur utilisation dans des compositions cosmétiques ou pharmaceutiques, notamment dermatologiques, à activité pigmentante.

Ainsi, selon un premier aspect, la présente invention fournit une composition à base de phases lamellaires lipidiques hydratées, ou de liposomes, de préférence pour la préparation de compositions cosmétiques ou pharmaceutiques, caractérisée en ce que lesdites phases lamellaires lipidiques hydratées ou lesdits liposomes contiennent au moins en partie, en tant que principe actif, de la tyrosine ou un dérivé de tyrosine, tel que par exemple un sel, ou un ester, à l'exclusion des sels de tyrosine avec au moins un acide aminé basique.

Dans la présente description et les revendications, le terme "lipidique" dans l'expression "phase lamellaire lipidique" couvre toutes les substances comprenant une chaîne carbonée dite grasse, généralement supérieure a 5 atomes de carbone.

Selon l'invention, on utilise des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques en présence d'une phase aqueuse. En particulier, citons parmi ces lipides : les phospholipides, les phosphoaminolipides, les glycolipides, les alcools gras polyoxyéthylénés, les esters de polyols éventuellement polyoxyéthylénés. De telles substances sont par exemple constituées par une lécithine d'oeuf ou de soja, une phosphatidylsérine, une sphyngomyéline, un cérébroside ou un stéarate de polyglycérol oxyéthyléné.

Plus particulièrement, la tyrosine est la L-tyrosine, et le dérivé de tyrosine précité est choisi parmi le groupe de composés constitué d'un L-tyrosinate alcalin ou alcalino-terreux comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+) glucose.

Comme on peut préparer différents composés de réaction à partir du D(+) glucose, tous ces composés sont inclus dans la portée de l'invention, pris seuls ou en mélange. Dans le commerce, on trouve un mélange de ces composés de réaction du D(+) glucose avec la L-tyrosine, en particulier un mélange d'esters, sous la dénomination "tyrosinate de glucose".

L'incorporation de la tyrosine ou de ses dérivés dans les phases lamellaires lipidiques hydratées ou dans les liposomes peut être effectuée selon des procédés connus. Ceux-ci sont choisis plus particulièrement en fonction du caractère plus ou moins lipophile ou plus ou moins hydrophile du composé à incorporer.

Selon un mode préféré de réalisation de la composition à base de phases lamellaires lipidiques hydratées ou de liposomes, on choisit une technique de préparation décrite dans le document EP-B1-0087993, éventuellement en combinaison avec une technique décrite dans le document EP-B1-0107559.

Ainsi, il est par exemple possible d'inclure la tyrosine ou le dérivé de tyrosine dans des phases lamellaires lipidiques hydratées ou des liposomes de la manière suivante :

### Etape 1

On dissout dans un solvant organique à point d'ébullition relativement bas, par exemple inférieur à 100°C à pression atmosphérique, tel que du dichlorométhane ou du méthanol, un lipide amphiphile, comme exemple de la lécithine de soja, hydrogénée ou non. On peut, en outre, dissoudre un lipide hydrophobe tel qu'un stérol, comme le cholestérol ou le β-sitostérol, et avantageusement un anti-oxydant comme l'α-tocophérol.

La quantité de lipide hydrophobe ne doit généralement pas être supérieure en poids à 0,2 fois la quantité de lipide amphiphile.

### Etape 2

On disperse ensuite dans la solution obtenue de la tyrosine ou le dérivé de tyrosine. Les proportions relatives de lipide d'une part et de la tyrosine ou du dérivé de tyrosine d'autre part sont, en poids, comprises entre 8 : 2 et 9,9 : 0,1. De préférence, le mélange est agité pendant 30 min à température ordinaire.

### Etape 3-A

Le mélange obtenu à la fin de la deuxième étape est introduit dans un ballon rotatif, et évaporé par chauffage au bain-marie, éventuellement sous pression réduite.

Après évaporation, le film lipidique déposé sur les parois est repris sous agitation par de l'eau ou par une solution aqueuse appropriée telle qu'une solution tampon contenant 0,8% de NaCl et 1,5% de NaH₂PO₄, dite "tampon phosphate".

De préférence, la quantité d'eau ou de solution aqueuse est au moins égale, en poids, à huit fois la quantité de lipide contenue dans le ballon.

On obtient ainsi une suspension de liposome, qui peut être ensuite homogénéisée par un moyen approprié, comme par exemple les ultrasons.

### Etape 3-B

Selon une variante du procédé de préparation des compositions de l'invention, on utilise le procédé décrit dans le document EP-B1-0087993, comprenant l'atomisation du mélange obtenu à la fin de la deuxième étape, suivi de la dispersion de la poudre lipidique ainsi obtenue dans une quantité prédéterminée d'eau ou d'une solution aqueuse de substances à encapsuler.

On obtient ainsi des phases lamellaires lipidiques hydratées ou une suspension de liposomes, selon que l'on a choisi de disperser la poudre lipidique dans peu ou beaucoup de milieu aqueux, ainsi qu'il est exposé dans le document européen précité.

La dispersion de phases lamellaires ou de liposomes peut ensuite être homogénéisée, par exemple selon le procédé décrit dans le document EP-B1-0107559.

### Etapes 3-C et 3-D

Selon une autre variante, on introduit la tyrosine ou le dérivé de tyrosine directement dans le milieu aqueux, dans lequel la phase lipidique sera dispersée.

Pour cela, on évapore la solution organique obtenue à la fin de l'étape 1, soit (étape 3-C) au moyen d'un ballon rotatif comme indiqué à l'étape 3-A, soit (étape 3-D) au moyen d'un atomiseur comme indiqué à l'étape 3-B. Le film lipidique ou la poudre lipidique respectivement obtenu est ensuite dispersé dans une solution aqueuse de tyrosine ou de dérivé de tyrosine.

Il se forme alors des phases lamellaires lipidiques ou des liposomes qui contiennent au moins en partie les substances dissoutes dans l'eau.

On peut ensuite homogénéiser par les procédés évoqués plus haut.

Avantageusement les dispersions de phases lamellaires lipidiques hydratées ou les suspensions de liposomes obtenues aux étapes 3 ci-dessus, peuvent être gélifiées, par exemple en les mélangeant avec un gel préparé séparément, comme un gel de polymère vinylique.

Selon un deuxième aspect, la présente invention concerne aussi une composition cosmétique ou pharmaceutique, notamment dermatologique, à activité pigmentante, caractérisée en ce qu'elle comprend une composition à base de phases lamellaires lipidiques hydratées ou de liposomes, telle que précédemment définie.

De préférence, la proportion en poids de tyrosine ou du dérivé de tyrosine est comprise entre 0,001% et 10%, de préférence 0,05% et 2% en poids, par rapport au poids total de ladite composition cosmétique ou pharmaceutique.

D'autres buts, caractéristiques et avantages de l'invention apparaîtront plus clairement à la lumière de la description explicative qui va suivre, faite en référence à plusieurs exemples. Dans ces exemples, les pourcentages sont donnés en poids, sauf indication contraire.

### Exemple 1 Préparation de phases lamellaires lipidiques hydratées contenant de la L-tyrosine (méthode A)

On dissout dans 20 ml de dichlorométhane 1,8 g de lécithine de soja hydrogénée et 0,06 g d'α-tocophérol, puis on disperse dans cette solution 0,2 g de L-tyrosine.

Ce mélange est évaporé sous pression réduite (200 mm de mercure environ) dans un ballon rotatif porté à 45°C.

Le film lipidique obtenu est repris par 98 g de la solution dite "tampon phosphate" précitée (pH environ égal à 7,5) sous agitation douce pendant 3 h.

On obtient après homogénéisation aux ultrasons (10 min, 100 W), une suspension aqueuse de liposomes.

Cette suspension est ensuite gélifiée en la mélangeant à poids égal avec un gel de Carbopol 94® préparé de façon classique à la concentration de 5% en poids.

On obtient ainsi environ 200 g d'une composition gélifiée à base de liposomes dont la teneur en tyrosine est de 0,1% en poids de cette composition.

### Exemple 2 Préparation de phases lamellaires lipidiques hydratées contenant de la L-tyrosine (Méthode B)

Dans 300 ml de dichlorométhane, on dissout 36 g de lécithine de soja, 0,5 g d'α-tocophérol, puis 4 g de β-sitostérol. La solution ainsi obtenue est atomisée à 65°C, comme décrit dans le brevet EP-B1-0087993, de façon à produire un mélange intime des deux constituants lipidiques sous forme d'une poudre fine.

On dissout par ailleurs 0,35 g de L-tyrosine dans 900 ml de la solution dite "tampon phosphate" précitée (pH environ égal à 7,5). La poudre lipidique atomisée est alors dispersée, au moyen d'un agitateur magnétique, pendant 3 h, dans la solution obtenue, puis on complète cette dispersion à 1000 g par de l'eau distillée, de manière à obtenir une suspension de liposomes de composition suivante :

| | |
|---|---|
| lécithine de soja | 36 g |
| β-sitostérol | 4 g |
| α-tocophérol | 0,5 g |
| L-tyrosine | 0,35 g |
| excipient aqueux qsp | 1 000 g |

On homogénéise cette composition au moyen d'un homogénéiseur sous pression, par exemple selon le procédé décrit dans le brevet EP-B1-0107559.

### Exemple 3 Préparation de phases lamellaires lipidiques hydratées contenant l'ester méthylique de la L-tyrosine

On opère comme dans l'exemple 1 avec 1,8 g de lécithine de soja hydrogénée, 0,2 g d'ester méthylique de L-tyrosine et 0,06 g d'α-tocophérol, que l'on dissout dans 80 ml de méthanol. Cette solution est évaporée sous pression réduite (200 mm de mercure environ) dans un ballon rotatif à 56°C environ. Le film lipidique est repris par 98 g de la solution dite "tampon phosphate" précitée (pH environ égal à 7,5). Sous agitation magnétique pendant 3 heures, on obtient, après homogénéisation aux ultrasons pendant 10 mn à 100 W, une suspension de liposomes contenant du L-tyrosinate de méthyle, que l'on peut ensuite gélifier avec gel de Carbopol 940®. La taille moyenne de liposomes, mesurée au Nano-Sizer® est de 85,2 nm.

### Exemple 4 Préparation de phases lamellaires lipidiques hydratées contenant du "tyrosinate de glucose"

On dilue dans 15 ml de méthanol 1,0 g d'une solution commerciale de "tyrosinate de glucose" (solution hydroalcoolique à 20%). On y ajoute 25 ml d'une solution de 3,8 g de lécithine de soja, de 0,2 g de β-sitostérol et 0,1 g d'α-tocophérol, dans du dichlorométhane.

La solution obtenue est atomisée à 75°C comme décrit dans le brevet européen EP-B1-0087993 de façon à produire 5 g de poudre du mélange lipidique. La poudre lipidique est dispersée dans 47,5 g d'eau déminéralisée, sous agitation modérée pendant 2 h. On obtient une suspension de phases lamellaires lipidiques que l'on homogénéise par ultrasons pendant 10 min à 100 W, dans un bain de glace.

Les 50 g de suspension homogénéisée de liposomes obtenue sont ensuite gélifiées par addition de 50 g d'un gel de Carbopol 940® à 1%.

La taille des liposomes, mesurée au Nano-Sizer® est de 145,5 ± 0,8 nm.

### Exemple 5 Mise en évidence de l'activité pigmentante de la tyrosine incluse dans des liposomes.

Pour étudier l'activité pigmentante de ces compositions selon l'invention, on a cherché à mettre en évidence l'augmentation de la mélanogénèse chez la souris in vivo.

Cette expérimentation a été effectuée sur des souris C₃H, par applications quotidiennes de produit à tester suivies d'une irradiation.

On utilise trois lots de 6 souris. Le premier lot (désigné ci-après "témoin non irradié") ne subit ni application de produit, ni irradiation aux U.V.

Le second lot (désigné ci-après "témoin irradié") ne subit aucune application de produit, mais une irradiation quotidienne aux U.V. 5 jours par semaine pendant trois semaines.

Enfin, le troisième lot subit une application quotidienne de produit à tester suivie d'une irradiation aux U.V., dans les mêmes conditions que le deuxième lot.

Pour réaliser cette expérimentation, on applique, sur la queue des souris, le produit obtenu sous forme de gel à l'exemple 2, à raison de 5 jours par semaine pendant trois semaines. Le produit est appliqué en une quantité d'environ 0,2 ml (variant selon la longueur de la queue) environ 1/4 d'heure avant l'irradiation.

Les souris des lots 2 et 3, lors de chaque irradiation, ont la queue exposée pendant 150 s à une distance de 26,5 cm, d'une lampe OSRAM® au xénon 2 500 W munie d'un filtre à eau, pour supprimer les grandes longueurs d'ondes et d'un filtre WG 320 pour filtrer les U.V.C. L'intensité électrique est réglée à 54 A, et les puissances d'irradiation un U.V.B. et U.V.A. sont respectivement 3 mW et 22 mW.

Les souris des trois lots sont ensuite sacrifiées, puis on prélève sur chacune d'elles, par biopsie, deux fragments de peau de queue que l'on immerge dans une solution de bromure de sodium 2 N, pendant 3 h à température ambiante pour séparer l'épiderme.

On détache les morceaux d'épiderme à l'aide de pinces, puis on les rince pendant 1 min à l'eau distillée, on les sèche et on les pèse.

Pour chaque souris, on pourra donc, avec le premier fragment, effectuer un dosage de la mélanine formée et, sur le deuxième fragment, effectuer une observation microscopique.

### A ― Dosage de la mélanine formée.

On soumet les fragments d'épiderme séchés et pesés à une digestion par la trypsine à 37°C pendant 48 h.

On effectue ensuite une filtration et une centrifugation à 3000 tr/min pendant 30 min.

On récupère le culot mélanique de chaque souris que l'on met en suspension dans de l'eau distillée en quantité nécessaire pour permettre la mesure de la densité optique.

La densité optique est mesurée de manière classique à 400 nm.

Les valeurs relevées sont ensuite ramenées à 100 mg de poids d'épiderme prélevé et on détermine une valeur moyenne de densité optique qui est seule prise en compte. Les résultats de ces mesures sont donnés au tableau I ci-après.

Ce tableau contient la mesure de la densité optique pour chaque souris, ainsi que la moyenne de ces densités pour chaque lot (valeurs soulignées).

Il apparaît très clairement que la quantité de mélanine formée, liée à la valeur de la densité optique, est beaucoup plus importante pour les souris du troisième lot qui ont reçu une application de la composition de l'exemple 2, à savoir une composition à base de liposomes contenant de la tyrosine, avant chaque irradiation.

### B ― Observation microscopique des fragments d'épiderme.

Par ces observation, on cherche à caractériser l'aspect des épidermes dans les trois lots de souris, en faisant apparaître en particulier les mélanocytes.

La visualisation des mélanocytes est effectuée selon la méthode dite par DOPA réaction, d'après la technique décrite par Renato J. STARICCO et Hermann PINKUS (Journal of Investigative Dermatology, 1956 pages 33-45).

Les fragments d'épiderme prélevés et traités comme indiqué plus haut sont placés en incubation dans une solution tamponnée de dl-dopa à 0,1% à 37°C pendant 1 h. Puis ce milieu d'incubation est renouvelé et l'incubation est poursuivie pendant 6 à 7 h.

Les épidermes sont ensuite fixés au formol à 10% pendant une nuit, puis déshydratés à l'alcool absolu, éclaircis au toluène et enfin montés entre lame et lamelle, pour être photographiés au grossissement X 475.

En annexe, on peut voir trois vues correspondant à chacun des lots avec la même numérotation et sélectionnées en fonction de leur représentativité.
- Vue 1: (témoin) : les mélanocytes sont assez petits, leurs dendrites sont peu nombreuses et courtes. Le fond est clair.
- Vue 2: (témoin + U.V.) : les mélanocytes présentent un plus grand nombre de dendrites que sur la Vue 1, certaines sont beaucoup plus longues, et le fond est un peu plus gris.
- Vue 3: (composition ex. 2 + U.V.) : les mélanocytes sont beaucoup plus gros que dans les deux vues précédentes, les dendrites sont à la fois nombreuses, plus longues et surtout plus épaisses. Le fond est gris, parsemé de grains foncés constitués par de la mélanine libérée.

Il apparaît donc à l'évidence que la composition à base de liposomes contenant de la tyrosine a provoqué chez la souris du lot 3 une très nette augmentation de l'activité des mélanocytes.

Cette expérimentation in vivo chez la souris met clairement en évidence l'activité stimulante des compositions selon l'invention sur la mélanogénèse, ainsi que leur intérêt pour la préparation de compositions cosmétiques ou dermatologiques à activité pigmentante.

**TABLEAU I**

| TEMOIN | TEMOIN + U.V. | Composition exemple 2 + U.V. |
|---|---|---|
| 275 | 335 | 494 |
| 221 | 682 | 826 |
| 381 | 520 | 955 |
| 224 | 624 | 433 |
| 293 | 588 | 545 |
| - | 492 | 423 |
| 278,8 | 540,1 | 612,6 |

### Exemple 6 Composition cosmétique sous forme gel

La composition selon l'exemple 1, peut être utilisée telle quelle comme composition cosmétique sous forme de gel.

Ce gel est appliqué quotidiennement pour préparer la peau au soleil. Ce traitement est pratiqué de préférence huit jours avant toute exposition au soleil.

### Exemple 7 Composition cosmétique solaire.

On prépare une suspension gélifiée de liposomes selon l'exemple 4 de composition suivante :

| | |
|---|---|
| lécithine de soja | 3,8 g |
| β-sitostérol | 0,2 g |
| α-tocophérol | 0,1 g |
| "tyrosinate de glucose" | 1 g |
| excipients aqueux gélifiés qsp | 100 g |

Cette suspension est mélangée à un volume égal d'une émulsion d'huile dans eau préparée séparément de façon classique et contenant un filtre U.V. tel que le Parsol MCX® à la concentration de 3%.

La composition obtenue peut être utilisée comme lait solaire bronzant.

### Exemple 8 Composition cosmétique sous forme de lotion.

On prépare une suspension gélifiée de liposomes selon la procédure décrite à l'exemple 1 en utilisant toutefois un gel de Carbopol 940® deux fois moins concentré met une composition définie comme suit :

| | |
|---|---|
| lécithine de soja hydrogéné | 9 g |
| L-tyrosine | 1 g |
| excipients aqueux gélifiés + parfums qsp | 100 g |

Cette lotion, appliquée matin et soir sur le cuir chevelu, ralentit l'apparition des cheveux gris.

### Exemple 9 Crème pour l'amélioration du teint

| | |
|---|---|
| lécithine de soja | 2 g |
| α-tocophérol | 0,06 g |
| L-tyrosine | 0,2 g |
| excipients pour émulsion huile dans l'eau qsp | 100 g |

On prépare séparément une suspension de liposomes selon l'exemple 1, d'une part, et une émulsion huile dans l'eau, d'autre part.

Ces deux préparations sont ensuite mélangées à raison d'un volume de suspension de liposomes pour trois volumes d'émulsion.

La crème obtenue appliquée quotidiennement sur le visage, de préférence le matin, lui redonne bonne mine.

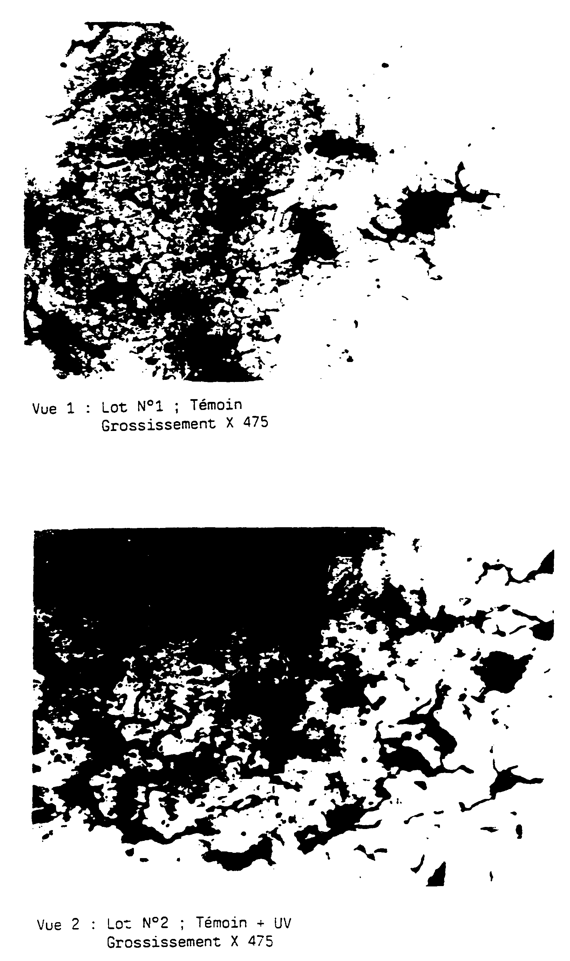

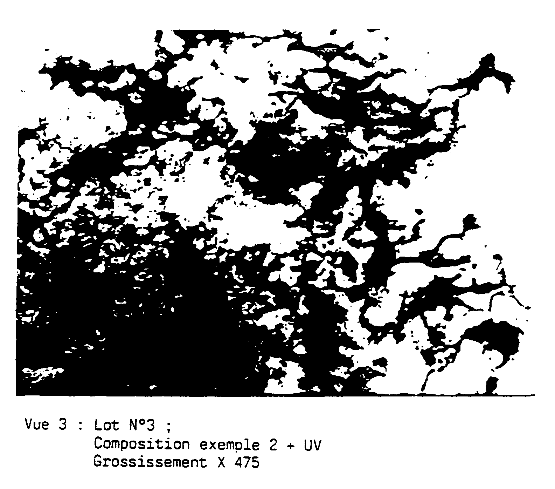

## Revendications (Revendications pour l'(les) Etat(s) contractant(s) suivant(s): DE, GB, IT, NL, SE, CH, LI, BE, AT, LU)

1. Composition à base de phases lamellaires lipidiques hydratées ou de liposomes contenant au moins en partie en tant que principe actif, de la tyrosine ou un dérivé de tyrosine, tel qu'un sel ou un ester, à l'exclusion des sels de tyrosine avec au moins un acide aminé basique, dans une proportion comprise entre 0,001% et 10% en poids par rapport au poids total de la composition.

2. Composition selon la revendication 1, caractérisée en ce que la proportion de tyrosine, ou de dérivé de tyrosine, est comprise entre 0,05% et 2% en poids par rapport au poids total de la composition.

3. Composition selon la revendication 1 ou 2, caractérisée en ce que la tyrosine est la L-tyrosine et le dérivé de tyrosine précité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

4. Composition selon l'une des revendications 1 à 3, caractérisée en ce que les phases lamellaires lipidiques hydratées ou les liposomes contiennent en outre du β-sitostérol.

5. Composition cosmétique ou pharmaceutique, notamment dermatologique, à activité pigmentante, caractérisée en ce qu'elle comprend une composition à base de phases lamellaires lipidiques hydratées ou de liposomes contenant, au moins en partie, en tant que principe actif, de la tyrosine ou un dérivé de tyrosine, tel qu'un sel ou un ester, à l'exclusion des sels de tyrosine avec au moins un acide aminé basique, dans une proportion comprise entre 0,001% et 10% en poids par rapport au poids total de la composition.

6. Composition selon la revendication 5, caractérisée en ce que la proportion de tyrosine, ou de dérivé de tyrosine, est comprise entre 0,05% et 2% en poids par rapport au poids total de la composition.

7. Composition selon la revendication 5 ou 6, caractérisée en ce que la tyrosine est la L-tyrosine et le dérivé de tyrosine précité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

8. Composition cosmétique ou pharmaceutique, notamment dermatologique, selon l'une quelconque des revendications 5 à 7, caractérisée en ce qu'elle contient en outre du β-sitostérol.

9. Utilisation cosmétique, comme agent pigmentant, de phases lamellaires lipidiques hydratées ou de liposomes contenant au moins en partie, en tant que principe actif, de la tyrosine ou un dérivé de tyrosine, tel qu'un sel ou un ester, à l'exclusion des sels de tyrosine avec au moins un acide aminé basique.

10. Utilisation selon la revendication 9, caractérisée en ce que la proportion de tyrosine, ou de dérivé de tyrosine, est comprise entre 0,001 % et 10%, de préférence 0,05% et 2% en poids par rapport au poids total de l'agent pigmentant.

11. Utilisation selon la revendication 9 ou 10, caractérisée en ce que la tyrosine est la L-tyrosine et la dérivé de tyrosine précité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

12. Utilisation selon l'une des revendications 9 à 11, caractérisée en ce que les phases lamellaires lipidiques hydratées ou les liposomes contiennent en outre du β-sitostérol.

## Revendications (Revendications pour l'(les) Etat(s) contractant(s) suivant(s): ES)

1. Procédé de préparation d'une composition à base de phases lamellaires lipidiques hydratées ou de liposomes comprenant l'incorporation au moins en partie, en tant que principe actif, dans lesdites phases lamellaires lipidiques hydratées ou lesdits liposomes, de la tyrosine ou un dérivé de tyrosine, tel qu'un sel ou un ester, à l'exclusion des sels de tyrosine avec au moins un acide aminé basique, dans une proportion comprise entre 0,001% et 10% en poids par rapport au poids total de la composition.

2. Procédé selon la revendication 1, caractérisé en ce que la proportion de tyrosine, ou de dérivé de tyrosine, est comprise entre 0,05% et 2% en poids par rapport au poids total de la composition.

3. Procédé selon la revendication 1 ou 2, caractérisé en ce que la tyrosine est la L-tyrosine et le dérivé de tyrosine précité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

4. Procédé selon l'une des revendications 1 à 3, caractérisé en ce que les phases lamellaires lipidiques hydratées ou les liposomes contiennent en outre du β-sitostérol.

5. Procédé de préparation d'une composition cosmétique ou pharmaceutique, notamment dermatologique, à activité pigmentante, caractérisé en ce qu'on utilise une composition à base de phases lamellaires lipidiques hydratées ou de liposomes contenant, au moins en partie, en tant que principe actif, de la tyrosine ou un dérivé de tyrosine, tel qu'un sel ou un ester, à l'exclusion des sels de tyrosine avec au moins un acide aminé basique, dans une proportion comprise entre 0,001% et 10% en poids par rapport au poids total de la composition.

6. Procédé selon la revendication 5, caractérisé en ce que la proportion de tyrosine, ou de dérivé de tyrosine, est comprise entre 0,05% et 2% en poids par rapport au poids total de la composition.

7. Procédé selon la revendication 5 ou 6, caractérisé en ce que la tyrosine est la L-tyrosine et le dérivé de tyrosine précité est choisi parmi le groupe de composés constitués d'un L-tyrosinate alcalin ou alcalino-terreux, comme par exemple le L-tyrosinate de sodium ou de calcium, du tyrosinate de méthyle, du L-tyrosinate d'éthyle, du L-tyrosinate de stéaryle et d'un composé obtenu à partir de L-tyrosine et de D(+)glucose.

8. Procédé selon l'une quelconque des revendications 5 à 7, caractérisé en ce qu'elle contient en outre du β-sitostérol.

## Revendications (Revendications pour l'(les) Etat(s) contractant(s) suivant(s): GR)

1. Composition à base de phases lamellaires lipidiques hydratées ou de liposomes contenant au moins en partie, en tant que principe actif, de la tyrosine ou un dérivé de tyrosine, tel qu'un sel ou un ester à l'exclusion des sels de tyrosine avec au moins un acide aminé basique, dans une proportion comprise entre 0,001 % et 10 % en poids par rapport au poids total de la composition.

2. Composition selon la revendication 1, caractérisée en ce que la proportion de tyrosine, ou de dérivé de tyrosine, est comprise entre 0,05 % et 2 % en poids par rapport au poids total de la composition.

3. Composition selon la revendication 1 ou 2, caractérisée en ce que la tyrosine est la L-tyrosine et le dérivé de tyrosine précité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

4. Composition selon l'une des revendications 1 à 3, caractérisée en ce que les phases lamellaires lipidiques hydratées ou les liposomes contiennent en outre du β-sitostérol.

5. Composition cosmétique ou pharmaceutique, notamment dermatologique, à activité pigmentante, caractérisée en ce qu'elle comprend une composition à base de phases lamellaires lipidiques hydratées ou de liposomes conteant, au moins en partie, en tant que principe actif, de la tyrosine ou un dérivé de tyrosine, tel qu'un sel ou un ester, à l'exclusion des sels de tyrosine avec au moins un acide aminé basique, dans une proportion comprise entre 0,001 % et 10 % en poids par rapport au poids total de la composition.

6. Composition selon la revendication 5, caractérisée en ce que la proportion de tyrosine, ou de dérivé de tyrosine, est comprise entre 0,05 % et 2 % en poids par rapport au poids total de la composition.

7. Composition selon la revendication 5 ou 6, caractérisée en ce que la tyrosine est la L-tyrosine et le dérivé de tyrosine pricité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

8. Composition cosmétique ou pharmaceutique, notamment dermatologique, selon l'une quelconque des revendications 5 à 7, caractérisée en ce qu'elle contient en outre du β-sitostérol.

9. Utilisation cosmétique, comme agent pigmentant, de phases lamellaires lipi-diques hydratées ou de liposomes contenant au moins en partie, en tant que principe actif, de la tyrosine ou un dérivé de tyrosine, tel qu'un sel ou un ester, à l'exclusion des sels de tyrosine avec au moins un acide aminé basique.

10. Utilisation selon la revendication 9, caractérisée en ce que la proportion de tyrosine, ou de dérivé de tyrosine, est comprise entre 0,001 % et 10%, de préférence 0,05% et 2% en poids par rapport au poids total de l'agent pigmentant.

11. Utilisation selon la revendication 9 ou 10, caractérisée en ce que la tyrosine est la L-tyrosine et le dérivé de tyrosine précité est choisi parmi le groupe de composés constitués d'un L-tyrosinate alcalin ou alcalino-terreux, comme par exemple le L-tyrosinate de sodium ou de calcium, du L-tyrosinate de méthyle, du L-tyrosinate d'éthyle, du L-tyrosinate de stéaryle et d'un composé obtenu à partir de L-tyrosine et de D(+)glucose.

12. Utilisation selon l'une des revendications 9 à 11, caractérisée en ce que les phases lamellaires lipidiques hydratées ou les liposomes contiennent en outre du β-sitostérol.

13. Procédé de préparation d'une composition à base de phases lamellaires lipidiques hydratées ou de liposomes, de préférence pour la préparation de compositions cosmétiques ou pharmaceutiques, caractérisé en ce qu'on incorpore une composition telle que définie à l'une quelconque des revendications 1 à 4.

## Claims (Claims for the following Contracting State(s): DE, GB, IT, NL, SE, CH, LI, BE, AT, LU)

1. Composition based on hydrated lipidic lamellar phases or on liposomes containing at least in part, as active ingredient, tyrosine or a tyrosine derivative, such as a salt or an ester, excluding tyrosine salts with at least one basic amino acid, in a proportion ranging between 0.001 % and 10 % by weight with respect to the total weight of the composition.

2. Composition according to claim 1, characterized in that the proportion of tyrosine, or of tyrosine derivative, ranges between 0.05 % and 2 % by weight with respect to the total weight of the composition.

3. Composition according to claim 1 or 2, characterized in that tyrosine is L-tyrosine and the tyrosine derivative is selected from the group consisting of compounds constituted of all alkaline or alkaline-earth L-tyrosinate, such as for example sodium or calcium L-tyrosinate, methyl L-tyrosinate, ethyl L-tyrosinate, stearyl L-tyrosinate and a compound obtained from L-tyrosine and D(+)glucose.

4. Composition according to claims 1 to 3, characterized in that the hydrated lipidic lamellar phases or the liposomes further contain β-sitosterol.

5. Cosmetic or pharmaceutical, notably dermatological composition, with pigmenting activity, characterized in that it comprises a composition based on hydrated lipidic lamellar phases or oil liposomes containing, at least in part, as active ingredient, tyrosine or a tyrosine derivative, such as a salt or an ester, excluding tyrosine salts with at least one basic amino acid, in a proportion ranging between 0.001 % and 10 % by weight with respect to the total weight of the composition.

6. Composition according to claim 5, characterized in that the proportion of tyrosine, or of a tyrosine derivative, ranges between 0.05 % and 2 % by weight with respect to the total weight of the composition.

7. Composition according to claim 5 or 6, characterized in that tyrosine is L-tyrosine and the tyrosine derivative is selected from the group consisting of compounds constituted of an alkaline or alkaline-earth L-tyrosinate, such as for example sodium or calcium L-tyrosinate, methyl L-tyrosinate, ethyl L-tyrosinate, stearyl L-tyrosinate, and of a compound obtained from L-tyrosine and from D(+)glucose.

8. Cosmetic or pharmaceutical, notably dermatological composition, according to any one of claims 5 to 7, characterized in that it further contains β-sitosterol.

9. Cosmetic use, as pigmenting agent, of hydrated lamellar lipidic phases or of liposomes containing at least in part, as active ingredient, tyrosine or a tyrosine derivative, such as a salt or an ester, excluding tyrosine salts with at least one basic amino acid.

10. Use according to claim 9, characterized in that the proportion of tyrosine, or of tyrosine derivative, ranges between 0.001 % and 10 %, preferably 0.05 % and 2 % by weight with respect to the total weight of the pigmenting agent.

11. Use according to claim 9 or 10, characterized in that tyrosine is L-tyrosine and the tyrosine derivative is selected among the group of compounds constituted of an alkaline or alkaline-earth L-tyrosinate, such as for example sodium or calcium L-tyrosinate, methyl L-tyrosinate, ethyl L-tyrosinate, stearyl L-tyrosinate and a compound obtained from L-tyrosine and from D(+)glucose.

12. Use according to one of claims 9 to 11, characterized in that the hydrated lamellar lipidic phases or the liposomes further contain β-sitosterol.

## Claims (Claims for the following Contracting State(s): ES)

1. Process of preparation of a composition based on hydrated lipidic lamellar phases or on liposomes comprising the incorporation of at least in part, as active ingredient, in the hydrated lipidic lamellar phases or in the liposomes, of tyrosine or a tyrosine derivative, such as a salt or an ester, excluding tyrosine salts with at least one basic amino acid, in a proportion ranging between 0.001 % and 10 % by weight with respect to the total weight of the composition.

2. Process according to claim 1, characterized in that the proportion of tyrosine, or of tyrosine derivative, ranges between 0.05 % and 2 % by weight with respect to the total weight of the composition.

3. Process according to claim 1 or 2, characterized in that tyrosine is L-tyrosine and the tyrosine derivative is selected from the group consisting of compounds constituted of an alkaline or alkaline-earth L-tyrosinate, such as for example sodium or calcium L-tyrosinate, methyl L-tyrosinate, ethyl L-tyrosinate, stearyl L-tyrosinate and a compound obtained from L-tyrosine and D(+)glucose.

4. Process according to claims 1 to 3, characterized in that the hydrated lipidic lamellar phases or the liposomes further contain β-sitosterol.

5. Process of preparation of a cosmetic or pharmaceutical, notably dermatological composition, with pigmenting activity, characterized in that it is used a composition based on hydrated lipidic lamellar phases or on liposomes containing, at least in part, as active ingredient, tyrosine or a tyrosine derivative, such as a salt or an ester, exclusing tyrosine salts with at least one basic amino acid, in a proportion ranging between 0.001 % and 10 % by weight with respect to the total weight of the composition.

6. Process according to claim 5, characterized in that the proportion of tyrosine, or of a tyrosine derivative, ranges between 0.05 % and 2 % by weight with respect to the total weight of the composition.

7. Process according to claim 5 or 6, characterized in that tyrosine is L-tyrosine and the tyrosine derivative is selected from the group consisting of compounds constituted of an alkaline or alkaline-earth L-tyrosinate, such as for example sodium or calcium L-tyrosinate, methyl L-tyrosinate, ethyl L-tyrosinate, stearyl L-tyrosinate, and of a compound obtained from L-tyrosine and from D(+)glucose.

8. Process according to any one of claims 5 to 7, characterized in that it further contains β-sitosterol.

## Claims (Claims for the following Contracting State(s): GR)

1. Composition based on hydrated lipidic lamellar phases or on liposomes containing at least in part, as active ingredient, tyrosine or a tyrosine derivative, such as a salt or an ester, excluding tyrosine salts with at least one basic amino acid, in a proportion ranging between 0.001 % and 10 % by weight with respect to the total weight of the composition.

2. Composition according to claim 1, characterized in that the proportion of tyrosine, or of tyrosine derivative, ranges between 0.05 % and 2 % by weight with respect to the total weight of the composition.

3. Composition according to claim 1 or 2, characterized in that tyrosine is L-tyrosine and the tyrosine derivative is selected from the group consisting of compounds constituted of an alkaline or alkaline-earth L-tyrosinate, such as for example sodium or calcium L-tyrosinate, methyl L-tyrosinate, ethyl L-tyrosinate, stearyl L-tyrosinate and a compound obtained from L-tyrosine and D(+)glucose.

4. Composition according to claims 1 to 3, characterized in that the hydrated lipidic lamellar phases or the liposomes further contain β-sitosterol.

5. Cosmetic or pharmaceutical, notably dermatological composition, with pigmenting activity, characterized in that it comprises a composition based on hydrated lipidic lamellar phases or on liposomes containing, at least in part, as active ingredient, tyrosine or a tyrosine derivative, such as a salt or an ester, excluding tyrosine salts with at least one basic amino acid, in a proportion ranging between 0.001 % and 10 % by weight with respect to the total weight of the composition.

6. Composition according to claim 5, characterized in that the proportion of tyrosine, or of a tyrosine derivative, ranges between 0.05 % and 2 % by weight with respect to the total weight of the composition.

7. Composition according to claim 5 or 6, characterized in that tyrosine is L-tyrosine and the tyrosine derivative is selected from the group consisting of compounds constituted of an alkaline or alkaline-earth L-tyrosinate, such as for example sodium or calcium L-tyrosinate, methyl L-tyrosinate, ethyl L-tyrosinate, stearyl L-tyrosinate, and of a compound obtained from L-tyrosine and from D(+)glucose.

8. Cosmetic or pharmaceutical, notably dermatological composition, according to any one of claims 5 to 7, characterized in that it further contains β-sistosterol.

9. Cosmetic use, as pigmenting agent, of hydrated lamellar lipidic phases or of liposomes containing at least in part, as active ingredient , tyrosine or a tyrosine derivative, such as salt or an ester, excluding tyrosine salts with at least one basic amino acid.

10. Use according to claim 9, characterized in that the proportion of tyrosine, or of tyrosine derivative, ranges between 0.001 % and 10 %, preferably 0.05 % and 2 % by weight with respect to the total weight of the pigmenting agent.

11. Use according to claim 9 or 10, characterized in that tyrosine is L-tyrosine and the tyrosine derivative is selected among the group of compounds constituted of an alkaline or alkaline-earth L-tyrosinate, such as for example sodium or calcium L-tyrosinate, methyl L-tyrosinate, ethyl L-tyrosinate, stearyl L-tyrosinate and a compound obtained from L-tyrosine and from D(+)glucose.

12. Use according to one of claims 9 to 11, characterized in that the hydrated lamellar lipidic phases or the liposomes further contain β-sitosterol.

13. Process of preparation of a composition based on hydrated lipidic lamellar phases or on liposomes, preferably for the preparation of cosmetic or pharmaceutical compositions, characterized in that it is incorporated a composition such as defined in any one of claims 1 to 4.

## Patentansprüche (Patentansprüche für folgende(n) Vertragsstaat(en): DE, GB, IT, NL, SE, CH, LI, BE, AT, LU)

1. Zusammensetzung auf der Basis von wasserhaltigen lamellaren Lipidphasen oder von Liposomen, die als Wirkstoff mindestens teilweise Tyrosin oder ein Tyrosinderivat, wie ein Salz oder einen Ester davon, in einem Mengenanteil von 0,001 bis 10 Gew.-%, bezogen auf das Gesamtgewicht der Zusammensetzung, enthält, wobei Salze des Tyrosins mit mindestens einer basischen Aminosäure ausgenommen sind.

2. Zusammensetzung nach Anspruch 1, dadurch gekennzeichnet, daß der Mengenanteil des Tyrosins oder des Tyrosinderivats 0,05 bis 2 Gew.-%, bezogen auf das Gesamtgewicht der Zusammensetzung, beträgt.

3. Zusammensetzung nach Anspruch 1 oder 2, dadurch gekennzeichnet, daß das Tyrosin L-Tyrosin ist und das Tyrosinderivat unter Alkali- und Erdalkali-L-tyrosinaten, wie beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

4. Zusammensetzung nach einem der Ansprüche 1 bis 3, dadurch gekennzeichnet, daß die wasserhaltigen lamellaren Lipidphasen oder die Liposomen ferner β-Sitosterin enthalten.

5. Kosmetische oder pharmazeutische und inbesondere dermatologische Zusammensetzung mit pigmentierender Wirkung,
dadurch gekennzeichnet, daß
sie eine Zusammensetzung auf der Basis von wasserhaltigen lamellaren Lipidphasen oder von Liposomen enthält, die als Wirkstoff mindestens teilweise Tyrosin oder ein Tyrosinderivat, wie ein Salz oder einen Ester davon, in einem Mengenanteil von 0,001 bis 10 Gew.-%, bezogen auf das Gesamtgewicht der Zusammensetzung, enthält, wobei Salze des Tyrosins mit mindestens einer basischen Aminosäure ausgenommen sind.

6. Zusammensetzung nach Anspruch 5, dadurch gekennzeichnet, daß der Mengenanteil des Tyrosins oder des Tyrosinderivats 0,05 bis 2 Gew.-%, bezogen auf das Gesamtgewicht der Zusammensetzung, beträgt.

7. Zusammensetzung nach Anspruch 5 oder 6, dadurch gekennzeichnet, daß das Tyrosin L-Tyrosin ist und das Tyrosinderivat unter Alkali- und Erdalkali-L-tyrosinaten, wie beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

8. Kosmetische oder pharmazeutische und insbesondere dermatologische Zusammensetzung nach einem der Ansprüche 5 bis 7, dadurch gegekennzeichnet, daß sie ferner β-Sitosterin enthält.

9. Kosmetische Verwendung von wasserhaltigen lamellaren Lipidphasen oder von Liposomen, die als Wirkstoff mindestens teilweise Tyrosin oder ein Tyrosinderivat, wie ein Salz oder einen Ester davon, enthalten, wobei Salze des Tyrosins mit mindestens einer basischen Aminosäure ausgenommen sind, als Pigmentierungsmittel.

10. Verwendung nach Anspruch 9, dadurch gekennzeichnet, daß der Mengenanteil des Tyrosins oder des Tyrosinderivats 0,001 bis 10 Gew.-% und vorzugsweise 0,05 bis 2 Gew.-%, bezogen auf das Gesamtgewicht des Pigmentierungsmittels, beträgt.

11. Verwendung nach Anspruch 9 oder 10, dadurch gekennzeichnet, daß das Tyrosin L-Tyrosin ist und das Tyrosinderivat unter Alkali- und Erdalkali-L-tyrosinaten, wei beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

12. Verwendung nach einem der Ansprüche 9 bis 11, dadurch gekennzeichnet, daß die wasserhaltigen lamellaren Lipidphasen oder die Liposomen ferner β-Sitosterin enthalten.

## Patentansprüche (Patentansprüche für folgende(n) Vertragsstaat(en): ES)

1. Verfahren zur Herstellung einer Zusammensetzung auf der Basis von wasserhaltigen lamellaren Lipidphasen oder von Liposomen, welches das Einbringen mindestens teilweise von Tyrosin oder einem Tyrosinderivat, wie eines Salzes oder eines Esters davon als Wirkstoff, in einem Mengenanteil von 0,001 bis 10 Gew.-%, bezogen auf das Gesamtgewicht der Zusammensetzung, umfaßt, wobei Salze des Tyrosins mit mindestens einer basischen Aminosäure ausgenommen sind.

2. Verfahren nach Anspruch 1, dadurch gekennzeichnet, daß der Mengenanteil des Tyrosins oder des Tyrosinderivats 0,05 bis 2 Gew.-%, bezogen auf das Gesamtgewicht der Zusammensetzung, beträgt.

3. Verfahren nach Anspruch 1 oder 2, dadurch gekennzeichnet, daß das Tyrosin L-Tyrosin ist und das Tyrosinderivat unter Alkali- und Erdalkali-L-tyrosinaten, wie beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

4. Verfahren nach einem der Ansprüche 1 bis 3, dadurch gekennzeichnet, daß die wasserhaltigen lamellaren Lipidphasen oder die Liposomen ferner β-Sitosterin enthalten.

5. Verfahren zur Herstellung einer kosmetischen oder pharmazeutischen und inbesondere dermatologischen Zusammensetzung mit pigmentierender Wirkung,
gekennzeichnet durch
Verwendung einer Zusammensetzung auf der Basis von wasserhaltigen lamellaren Lipidphasen oder von Liposomen, die als Wirkstoff mindestens teilweise Tyrosin oder ein Tyrosinderivat, wie ein Salz oder einen Ester davon, in einem Mengenanteil von 0,001 bis 10 Gew.-%, bezogen auf das Gesamtgewicht der Zusammensetzung, enthält, wobei Salze des Tyrosins mit mindestens einer basischen Aminosäure ausgenommen sind.

6. Verfahren nach Anspruch 5, dadurch gekennzeichnet, daß der Mengenanteil des Tyrosins oder des Tyrosinderivats 0,05 bis 2 Gew.-%, bezogen auf das Gesamtgewicht der Zusammensetzung, beträgt.

7. Verfahren nach Anspruch 5 oder 6, dadurch gekennzeichnet, daß das Tyrosin L-Tyrosin ist und das Tyrosinderivat unter Alkali- und Erdalkali-L-tyrosinaten, wie beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

8. Verfahren nach einem der Ansprüche 5 bis 7, dadurch gegekennzeichnet, daß die Zusammensetzung ferner β-Sitosterin enthält.

## Patentansprüche (Patentansprüche für folgende(n) Vertragsstaat(en): GR)

1. Zusammensetzung auf der Basis von wasserhaltigen lamellaren Lipidphasen oder von Liposomen, die als Wirkstoff mindestens teilweise Tyrosin oder einem Tyrosinderivat, wie ein Salz oder einen Ester davon, in einem Mengenanteil von 0,001 bis 10 Gew.-%, bezogen auf das Gesamtgewicht der Zusammensetzung, enthält, wobei Salze des Tyrosins mit mindestens einer basischen Aminosäure ausgenommen sind.

2. Zusammensetzung nach Anspruch 1, dadurch gekennzeichnet, daß der Mengenanteil des Tyrosins oder des Tyrosinderivats 0,05 bis 2 Gew.-%, bezogen auf das Gesamtgewicht der Zusammensetzung, beträgt.

3. Zusammensetzung nach Anspruch 1 oder 2, dadurch gekennzeichnet, daß das Tyrosin L-Tyrosin ist und das Tyrosinderivat unter Alkali- und Erdalkali-L-tyrosinaten, wie beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

4. Zusammensetzung nach einem der Ansprüche 1 bis 3, dadurch gekennzeichnet, daß die wasserhaltigen lamellaren Lipidphasen oder die Liposomen ferner β-Sitosterin enthalten.

5. Kosmetische oder pharmazeutische und inbesondere dermatologische Zusammensetzung mit pigmentierender Wirkung,
dadurch gekennzeichnet, daß
sie eine Zusammensetzung auf der Basis von wasserhaltigen lamellaren Lipidphasen oder von Liposomen enthält, die als Wirkstoff mindestens teilweise Tyrosin oder ein Tyrosinderivat, wie ein Salz oder einen Ester davon, in einem Mengenanteil von 0,001 bis 10 Gew.-%, bezogen auf das Gesamtgewicht der Zusammensetzung, enthält, wobei Salze des Tyrosins mit mindestens einer basischen Aminosäure ausgenommen sind.

6. Zusammensetzung nach Anspruch 5, dadurch gekennzeichnet, daß der Mengenanteil des Tyrosins oder des Tyrosinderivats 0,05 bis 2 Gew.-%, bezogen auf das Gesamtgewicht der Zusammensetzung, beträgt.

7. Zusammensetzung nach Anspruch 5 oder 6, dadurch gekennzeichnet, daß das Tyrosin L-Tyrosin ist und das Tyrosinderivat unter Alkali- und Erdalkali-L-tyrosinaten, wie beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

8. Kosmetische oder pharmazeutische und insbesondere dermatologische Zusammensetzung nach einem der Ansprüche 5 bis 7, dadurch gegekennzeichnet, daß sie ferner β-Sitosterin enthält.

9. Kosmetische Verwendung von wasserhaltigen lamellaren Lipidphasen oder von Liposomen, die als Wirkstoff mindestens teilweise Tyrosin oder ein Tyrosinderivat, wie ein Salz oder einen Ester davon, enthalten, wobei Salze des Tyrosins mit mindestens einer basischen Aminosäure ausgenommen sind, als Pigmentierungsmittel.

10. Verwendung nach Anspruch 9, dadurch gekennzeichnet, daß der Mengenanteil des Tyrosins oder des Tyrosinderivats 0,001 bis 10 Gew.-% und vorzugsweise 0,05 bis 2 Gew.-%, bezogen auf das Gesamtgewicht des Pigmentierungsmittels, beträgt.

11. Verwendung nach Anspruch 9 oder 10, dadurch gekennzeichnet, daß das Tyrosin L-Tyrosin ist und das Tyrosinderivat unter Alkali- und Erdalkali-L-tyrosinaten, wei beispielsweise Natrium- und Calcium-L-tyrosinat, Methyl-L-tyrosinat, Ethyl-L-tyrosinat, Stearyl-L-tyrosinat und aus L-Tyrosin und D(+)-Glucose erhaltenen Verbindungen ausgewählt ist.

12. Verwendung nach einem der Ansprüche 9 bis 11, dadurch gekennzeichnet, daß die wasserhaltigen lamellaren Lipidphasen oder die Liposomen ferner β-Sitosterin enthalten.

13. Verfahren zur Herstellung einer Zusammensetzung auf der Basis von wasserhaltigen lamellaren Lipidphasen oder von Liposomen, vorzugsweise zur Herstellung von kosmetischen oder pharmazeutischen Zusammensetzungen,
gekennzeichnet durch
Einbringen einer Zusammensetzung nach einem der Ansprüche 1 bis 4.
